# FASCICULE DE BREVET EUROPEEN

(11) **EP 0 570 050 B1**
(45) Date de publication et mention de la délivrance du brevet: **07.01.1998**
(21) Numéro de dépôt: 93201259.4
(22) Date de dépôt: 04.05.1993
(51) Int. Cl.: C07C 17/00, C07C 1/26, C07C 19/04

(54) **Procédé de déchloration des chlorométhanes supérieurs**
Verfahren zur Dechlorierung von höheren Chlormethanen
Process for the dechlorination of higher chloromethanes

(30) Priorité: 14.05.1992 BE 9200444
(43) Date de publication de la demande: 18.11.1993
(73) Titulaire: SOLVAY (Société Anonyme), B-1050 Bruxelles (BE)
(72) Inventeur: Schoebrechts, Jean-Paul, B-1390 Grez-Doiceau (BE); Janssens, Francine, B-1800 Vilvoorde (BE)
(74) Mandataire: Jacques, Philippe

(56) Documents cités:
- EP-A- 0 455 547
- US-A- 3 579 596

## Description

L'invention concerne un procédé de déchloration des chlorométhanes supérieurs, c'est-à-dire du chloroforme (CHCl₃) et du tétrachlorure de carbone (CCl₄), en phase gazeuse, par réaction avec de l'hydrogène.

Le brevet US-A-3,579,596 de DOW décrit la déchloration du tétrachlorure de carbone et du chloroforme par réaction avec de l'hydrogène en phase gazeuse en présence d'un catalyseur constitué d'un métal choisi parmi les métaux Ru, Rh, Pd, Os, Ir, Pt, Cu, Ag ou Au, utilisé indifféremment tel quel ou déposé sur un support tel que l'alumine, le carbone ou la silice. Dans les exemples de ce brevet, on illustre la mise en oeuvre d'un catalyseur constitué de 0,5 % de platine sur une alumine de caractéristiques non précisées. En reproduisant ces exemples, on constate une forte irreproductibilité des résultats et, dans bien des cas, une chute rapide de l'activité du catalyseur.

Selon la demande de brevet EP-A-0455547 de ATOCHEM, il est possible d'éviter l'instabilité thermique de la réaction et l'encrassement du catalyseur, qui serait responsable de sa rapide désactivation, en effectuant la réaction en présence d'oxygène. Une telle solution présente cependant plusieurs inconvénients pratiques majeurs. L'introduction simultanée d'oxygène et d'hydrogène est potentiellement dangereuse. L'augmentation de température qui résulte de l'introduction d'oxygène favorise la formation de méthane, réaction fortement exothermique qui augmente les risques d'emballement thermique du réacteur. La formation simultanée d'eau et de chlorure d'hydrogène génère dans le réacteur un mélange de produits particulièrement corrosif.

L'invention vise à procurer un procédé de déchloration des chlorométhanes supérieurs en phase gazeuse, qui ne présente aucun des inconvénients des procédés de l'art antérieur et qui, en particulier, assure une stabilité catalytique élevée, sans devoir recourir à un procédé nécessitant la mise en oeuvre simultanée d'oxygène et d'hydrogène.

La présente invention concerne donc un procédé de déchloration de chlorométhanes supérieurs en phase gazeuse, à l'aide d'hydrogène, en présence d'un catalyseur d'hydrogénation constitué essentiellement d'au moins un métal choisi parmi les métaux du groupe VIII et du groupe IB du tableau périodique des éléments déposé sur une alumine, caractérisé en ce que l'alumine possède une surface spécifique inférieure à environ 100 m²/g et en ce que la déchloration est réalisée en absence substantielle d'oxygène.

Le procédé de l'invention permet de déchlorer du tétrachlorure de carbone en chloroforme et en méthane ou du chloroforme en chlorure de méthylène et en méthane sans que l'on observe de désactivation rapide du catalyseur, bien que la déchloration s'effectue sans introduction d'oxygène dans le réacteur. Le procédé de l'invention est particulièrement performant lorsqu' il est appliqué à la déchloration du tétrachlorure de carbone.

L'alumine utilisée code support de catalyseur dans le procédé de l'invention peut être de toute origine et peut être obtenue par tout procédé connu, pour autant que sa surface spécifique, mesurée par physisorption de l'azote selon la méthode B.E.T., soit inférieure à environ 100 m²/g. De préférence, l'alumine utilisée dans le procédé selon l'invention possède une surface spécifique inférieure ou égale à environ 90 m²/g. De manière tout particulièrement préférée, sa surface spécifique ne dépasse pas environ 60 m²/g. L'alumine utilisée peut posséder une très faible surface spécifique, par exemple de l'ordre de 1 à 3 m²/g. On utilise de préférence une alumine dont la surface spécifique est d'au moins environ 5 m²/g.

Les caractéristiques classiques des alumines et leur mode habituel de préparation sont décrits, par exemple, dans "Encyclopedia of Chemical Technology", KIRK-OTHMER, 1978, 3ème édition, Vol. 2, p. 218 à 234. Elles sont généralement préparées par calcination à une température appropriée d'un hydroxyde d'aluminium ou d'un oxyde d'aluminium hydraté. Généralement, plus la température de calcination est élevée, plus la surface spécifique de l'alumine obtenue est faible. Selon la nature du composé précurseur et les conditions de calcination, des alumines de différentes variétés cristallographiques sont obtenues.

Des alumines de transition, notamment celles de phase cristalline κ, θ ou δ, qui peuvent être obtenues généralement par calcination des composés précurseurs à une température de l'ordre de 800 °C ou plus, ainsi que l'alumine α, obtenue généralement par calcination à une température de l'ordre de 1100 °C ou plus, ont conduit à de bons résultats dans le procédé de l'invention, à condition que leur surface spécifique soit inférieure à environ 100 m²/g. Très fréquemment, de telles alumines renferment plusieurs variétés cristallines.

L'alumine présente généralement un volume poreux d'environ 0,1 à environ 1 cm³/g. De préférence, il est de 0,2 à 0,8 cm³/g. Ces valeurs de volume poreux sont mesurées par porosimétrie au mercure pour les alumines macroporeuses ayant des pores de rayon supérieur à 75 Å et par physisorption d'azote dans tous les autres cas.

Selon son origine, l'alumine utilisée peut contenir de faibles quantités d'impuretés, principalement divers oxydes métalliques, tels que notamment des oxydes de sodium, de fer, de titane et de silicium. Généralement, on met en oeuvre code support une alumine dont la teneur totale en oxydes métalliques autres que Al₂O₃ ne dépasse pas 2 % et, de préférence pas 1 %.

En ce qui concerne spécifiquement la teneur en silice, on préfère une alumine contenant moins d'environ 0,1 % en poids de silice (SiO₂) et, plus particulièrement, une alumine dont la teneur pondérale en silice est inférieure à environ 0,05 %.

A titre d'exemples non limitatifs de métaux du groupe VIII ou du groupe IB du tableau périodique des éléments utilisables pour former le catalyseur d'hydrogénation déposé sur une alumine selon l'invention, on peut mentionner le ruthénium, le rhodium, le palladium, l'osmium, l'iridium, le platine, le cuivre, l'argent et l'or et leurs mélanges. De préférence, le métal utilisé est le platine ou le palladium. De bons résultats ont été obtenus avec le platine.

Généralement, la teneur en métal est d'environ 0,01 à environ 5 % du poids total du catalyseur, c'est-à-dire de la soie des poids de l'alumine et du métal. De préférence, cette teneur est d'environ 0,1 à environ 2 %. De bons résultats ont été obtenus avec une teneur en métal d'environ 0,3 % à environ 1 %.

Le mode d'obtention des catalyseurs utilisables dans le procédé selon l'invention n'est pas critique. Classiquement, les catalyseurs peuvent être obtenus par imprégnation de l'alumine avec un composé du métal, lequel est ensuite réduit à l'état métallique, par exemple par calcination en présence d'hydrogène.

Une préparation ayant donné de bons résultats consiste à mettre en contact sous vide l'alumine et une solution acide d'un composé du métal. Par exemple, lorsque l'on souhaite obtenir un catalyseur au platine, on peut opérer en présence d'acide chloroplatinique hexahydraté dissous dans une solution aqueuse d'acide chlorhydrique.

Avant l'emploi, le catalyseur est activé de manière classique. Par exemple, une activation qui a donné de bons résultats consiste à balayer la composition catalytique successivement sous air ou sous oxygène, d'abord à une température de 100 à 150 °C puis à une température de 300 à 550 °C, ensuite sous gaz inerte tel que l'azote, à une température de 75 à 125 °C, puis sous hydrogène, à une température de 200 à 300 °C et enfin sous gaz inerte tel que l'azote à température ambiante.

En raison, d'une part, du caractère très doux du traitement d'activation du catalyseur, effectué généralement à des températures nettement inférieures à la température de préparation de l'alumine utilisée dans le procédé selon l'invention et, d'autre part, de la quantité modérée de composé métallique déposé sur l'alumine, le catalyseur présente habituellement une surface spécifique et une porosité quasi identiques à celles de l'alumine utilisée comme support.

La température à laquelle s'effectue la réaction, se situe habituellement d'environ 50 °C à environ 200 °C. De préférence, cette température est de 70 à 180 °C. Lorsque le procédé de l'invention est appliqué à la déchloration du tétrachlorure de carbone, de bons résultats ont été obtenus pour la fabrication sélective de chloroforme à une température réactionnelle située de 80 à 160 °C.

La pression à laquelle est effectuée la réaction, se situe généralement d'environ 0,1 bar à environ 10 bar. De préférence, elle se situe de 1 à 8 bar. De bons résultats ont été obtenus avec une pression de 1 à 6 bar.

Le rapport molaire entre l'hydrogène et le chlorométhane supérieur mis en oeuvre est généralement d'environ 1 à environ 16. De préférence, ce rapport est d'environ 2 à environ 14. Lorsque le procédé de l'invention est appliqué à la déchloration du tétrachlorure de carbone, des rapports molaires situés dans cette plage favorisent la sélectivité en chloroforme. On a aussi observé que la dilution de la phase gazeuse par de l'hydrogène ralentit la formation de dépôts carbonés sur la composition catalytique et renforce donc la bonne tenue dans le temps des catalyseurs. C'est pourquoi ce rapport est de préférence d'au moins 4. De très bons résultats ont été obtenus lorsque ce rapport ne dépasse pas 12.

Les réactifs, hydrogène et chlorométhane supérieur, sont par ailleurs avantageusement dilués par du méthane qui ralentit également le vieillissement de la composition catalytique. Ainsi, le procédé selon l'invention peut être mis en oeuvre en présence de méthane. Dans ce cas, le rapport molaire entre le méthane et le chlorométhane supérieur mis en oeuvre est généralement d'environ 0,1 à environ 15. De préférence, ce rapport est d'environ 3 à environ 12. Lorsque le procédé de l'invention est appliqué à la déchloration du tétrachlorure de carbone, de bons résultats ont été obtenus avec un rapport d'environ 4 à environ 10. Le méthane mis en oeuvre est avantageusement le méthane coproduit dans la réaction de déchloration.

Le procédé selon l'invention est réalisé en l'absence substantielle d'oxygène. On n'introduit pas volontairement d'oxygène dans le réacteur et on veille à ce que la teneur en oxygène soit en général inférieure à 0,005 % du volume total des réactifs alimentant le réacteur, de préférence inférieure à 0,002 % du volume total des réactifs.

Après séparation des produits de réaction, le mélange gazeux d'hydrogène et de méthane peut avantageusement être recyclé au réacteur.

Le temps de contact moyen, c'est-à-dire le rapport entre le volume de la partie du réacteur occupée par le lit catalytique et le débit des réactifs à la pression et à la température de la réaction, est généralement d'environ 1 s à environ 30 s. Habituellement, il est d'environ 2 s à environ 15 s. Lorsque le procédé de l'invention est appliqué à la déchloration du tétrachlorure de carbone, de bons résultats ont été obtenus avec un temps de contact d'environ 3 s à environ 10 s.

On réalise le procédé selon l'invention en phase gazeuse, avec un catalyseur disposé en lit fixe ou en lit fluidisé, dans tout appareillage ou réacteur permettant de réunir les conditions de réaction décrites et permettant d'évacuer suffisamment la chaleur générée par la réaction pour éviter un emballement du réacteur.

Les catalyseurs utilisés dans le procédé de l'invention présentent de nombreux avantages, dont principalement une stabilité nettement supérieure à celle des catalyseurs de l'art antérieur. De plus, lorsque le procédé est appliqué à la déchloration du tétrachlorure de carbone, on observe une augmentation de la sélectivité en chloroforme au cours du temps, à l'inverse de l'effet généralement observé avec les catalyseurs de l'art antérieur. Conjugué à la très bonne stabilité des catalyseurs, cet effet entraîne une hausse du rendement en chloroforme (fraction de tétrachlorure de carbone mis en oeuvre transformée en chloroforme) au cours du temps. Les catalyseurs utilisés dans le procédé de l'invention induisent en outre la formation de quantités nettement plus faibles de produits en C₂, tels que notamment le perchloroéthylène et l'hexachloroéthane. Ils peuvent par ailleurs être aisément régénérés, sans perte notable d'activité et de sélectivité. Un procédé de régénération ayant donné de bons résultats consiste à appliquer au catalyseur deux traitements (balayage) par l'air à une température d'environ 350 à environ 500 °C pendant une durée suffisante pour éliminer les composés carbonés déposés à la surface du catalyseur, par exemple pendant 3 à 50 h, chacun suivi d'une réduction sous hydrogène, par exemple d'environ 250 °C à environ 350 °C, pendant environ 3 à 10 heures.

L'invention se trouve plus amplement illustrée par les exemples suivants. Les exemples 1 à 4 sont réalisés selon l'invention. Les exemples 5R et 6R sont donnés à titre de comparaison.

### Exemple 1

Un échantillon de catalyseur est préparé au départ d'une alumine α dont les caractéristiques sont les suivantes :
- surface spécifique B.E.T. = 8 m²/g;
- volume poreux (porosimétrie au mercure) = 0,39 cm³/g;
- teneur en Na₂O = 1,7 g/kg;
- teneur en SiO₂ = 0,27 g/kg;
- teneur en Fe₂O₃ = 0,09 g/kg;
- teneur en TiO₂ = 0,24 g/kg.

On imprègne cette alumine à température ambiante, à une pression d'environ 30 mbar, par une solution aqueuse d'acide hexachloroplatinique, en quantité appropriée pour obtenir, après séchage et activation, un catalyseur contenant 0,5 % en poids de platine.

5 cm³ de catalyseur, dilués par un volume égal de billes de verre, sont disposés en lit fixe dans un réacteur tubulaire en acier inoxydable, de diamètre intérieur de 10,2 mm, muni d'une gaine centrale de 1,6 mm de diamètre au sein de laquelle est disposé un thermocouple permettant de mesurer la température effective du lit catalytique. Le réacteur est chauffé au moyen d'un four à air pulsé, à une température de consigne donnée, régulée indépendamment de la température du lit. La température de consigne est la température de l'air environnant le réacteur. Les billes de verre sont utilisées dans un tel appareillage de laboratoire pour permettre un meilleur contrôle de l'exothermicité de la réaction. Les échanges calorifiques sont cependant insuffisants pour éviter toute apparition d'un profil thermique dans le lit catalytique.

On alimente le réacteur par un mélange gazeux constitué d'hydrogène et de tétrachlorure de carbone, avec un rapport molaire H₂/CCl₄ de 5, sous une pression de 4 bar. Les débits sont ajustés pour obtenir un temps de contact de 7 s à la température de consigne. Celle-ci est réglée de manière à obtenir un taux de conversion initial du tétrachlorure de carbone d'environ 95 %, taux atteint dans ces conditions avec ce catalyseur pour une température de consigne de 80 °C. Les produits de réaction sont analysés par chromatographie en phase gazeuse. Outre le tétrachlorure de carbone, les produits de réaction identifiés sont le chloroforme (CHCl₃), le dichlorométhane (CH₂Cl₂), le monochlorométhane (CH₃Cl), le méthane (CH₄), le perchloroéthylène (C₂Cl₄), le trichloroéthylène (C₂HCl₃) et l'hexachloroéthane (C₂Cl₆). Les sélectivités en ces différents produits sont exprimées en pourcentage volumique du tétrachlorure de carbone converti. Le solde du tétrachlorure de carbone transformé est constitué de produits non identifiés.

Les résultats obtenus après 1 heure, après 40 heures et après 118 heures de réaction sont rassemblés au tableau 1.

### Exemple 2

Un échantillon de catalyseur est préparé au départ d'une alumine dont les caractéristiques sont les suivantes :
- phase cristalline majeure : κ;
- phase cristalline mineure : α;
- surface spécifique B.E.T. = 56 m²/g;
- volume poreux (porosimétrie par adsorption d'azote) = 0,29 cm³/g;

On effectue le test catalytique dans les mêmes conditions que dans l'exemple 1, mais à une température de consigne de 95 °C, de manière à obtenir un taux de conversion du tétrachlorure de carbone d'environ 95 %.

Les résultats obtenus après 2 heures 15, après 16 heures 15 et après 137 heures de réaction sont également présentés au tableau 1.

### Exemple 3

Un échantillon de catalyseur est préparé comme dans l'exemple 1, au départ d'une alumine dont les caractéristiques sont les suivantes :
- phase cristalline majeure : θ;
- phases cristallines mineures : δ et α;
- surface spécifique B.E.T. = 84 m²/g;
- volume poreux (porosimétrie par adsorption d'azote) = 0,49 cm³/g;
- teneur en Na₂O < 0,03 g/kg;
- teneur en SiO₂ = 0,12 g/kg;
- teneur en Fe₂O₃ = 0,05 g/kg;
- teneur en TiO₂ = 1,1 g/kg.

On effectue le test catalytique dans les mêmes conditions que dans l'exemple 1.

Les résultats obtenus après 1 heure, après 23 heures et après 94 heures de réaction sont également présentés au tableau 1.

### Exemple 4

Un échantillon de catalyseur est préparé code dans l'exemple 1, au départ d'une alumine dont les caractéristiques sont les suivantes :
- phase cristalline majeure : δ;
- phases cristallines mineures : γ et η;
- surface spécifique B.E.T. = 89 m²/g;
- volume poreux (porosimétrie par adsorption d'azote) = 0,68 cm³/g;
- teneur en Na₂O = 0,45 g/kg;
- teneur en SiO₂ = 0,10 g/kg;
- teneur en Fe₂O₃ = 1,2 g/kg;
- teneur en TiO₂ < 0,02 g/kg.

On effectue le test catalytique dans les mêmes conditions que dans l'exemple 1, mais à une température de consigne de 110 °C, de manière à obtenir un taux de conversion du tétrachlorure de carbone d'environ 95 %.

Les résultats obtenus après 3 heures, après 19 heures et après 90 heures de réaction sont également présentés au tableau 1.

### Exemple 5R

Un échantillon de catalyseur est préparé comme dans l'exemple 1, au départ d'une alumine dont les caractéristiques sont les suivantes :
- phase cristalline majeure : θ;
- phase cristalline mineure : δ;
- surface spécifique B.E.T. = 133 m²/g;
- volume poreux (porosimétrie par adsorption d'azote) = 0,52 cm³/g;
- teneur en Na₂O = 0,03 g/kg;
- teneur en SiO₂ = 0,11 g/kg;
- teneur en Fe₂O₃ = 0,08 g/kg;
- teneur en TiO₂ = 0,75 g/kg.

On effectue le test catalytique dans les mêmes conditions que dans l'exemple 1.

Les résultats obtenus après 3 heures 15 de réaction et après 24 heures de réaction sont également présentés au tableau 1.

### Exemple 6R

Un échantillon de catalyseur est préparé comme dans l'exemple 1, au départ d'une alumine dont les caractéristiques sont les suivantes :
- phases cristallines majeures : γ et η;
- phase cristalline mineure : χ;
- surface spécifique B.E.T. = 239 m²/g;
- volume poreux (porosimétrie par adsorption d'azote) = 0,39 cm³/g;
- teneur en Na₂O = 3,1 g/kg;
- teneur en SiO₂ = 0,17 g/kg;
- teneur en Fe₂O₃ = 0,12 g/kg;
- teneur en TiO₂ < 0,02 g/kg.

On effectue le test catalytique dans les mêmes conditions que dans l'exemple 1, mais à une température de consigne de 90 °C, de manière à obtenir un taux de conversion du tétrachlorure de carbone d'environ 95 %.

Les résultats obtenus après 1 heure 30 de réaction et après 20 heures de réaction sont également présentés au tableau 1.

La comparaison des exemples 1 à 4 avec les exemples de comparaison 5R et 6R montre à suffisance la stabilité catalytique nettement supérieure des catalyseurs selon l'invention en ce qui concerne le taux de conversion, ainsi que les sélectivités plus élevées en chloroforme et plus faibles en méthane et en produits en C₂.

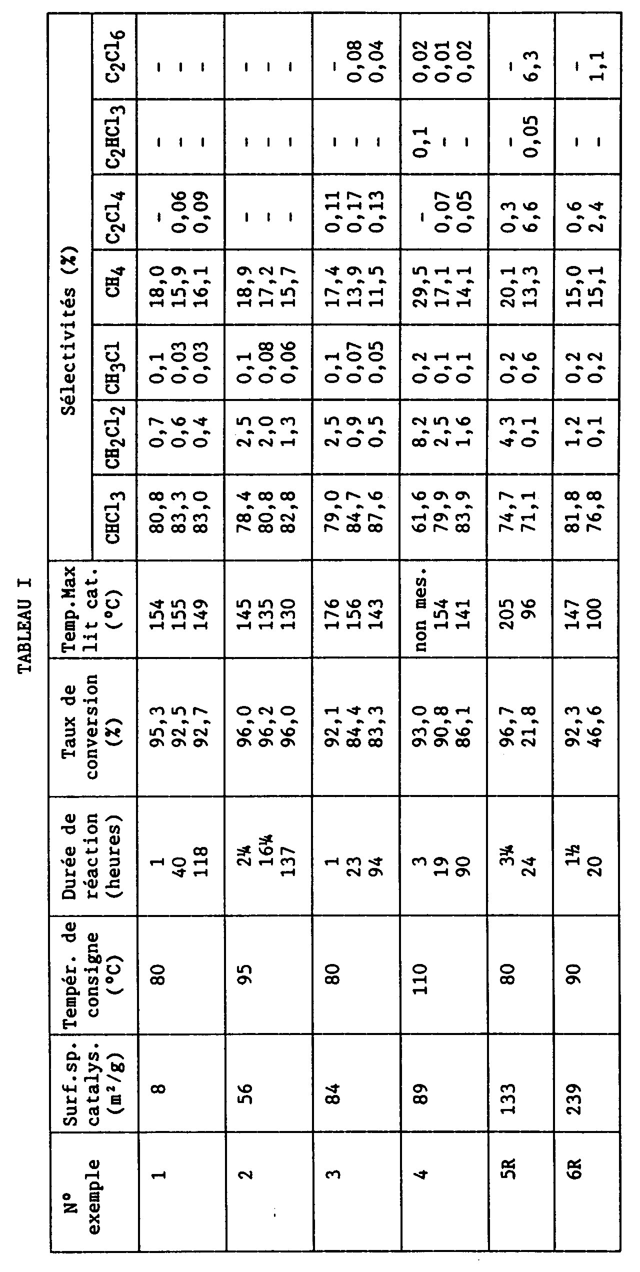

## Revendications

1. Procédé de déchloration de chlorométhanes supérieurs en phase gazeuse, à l'aide d'hydrogène, en présence d'un catalyseur d'hydrogénation constitué essentiellement d'au moins un métal choisi parmi les métaux du groupe VIII et du groupe IB du tableau périodique des éléments déposé sur une alumine, caractérisé en ce que l'alumine possède une surface spécifique inférieure à 100 m²/g et en ce que la déchloration est réalisée en absence substantielle d'oxygène.

2. Procédé selon la revendication 1, caractérisé en ce que la surface spécifique de l'alumine est de 5 m²/g à 90 m²/g.

3. Procédé selon la revendication 1 ou 2, caractérisé en ce que l'alumine ne contient pas plus de 2 % en poids d'oxydes métalliques autres que Al₂O₃.

4. Procédé selon l'une quelconque des revendications 1 à 3, caractérisé en ce que l'alumine contient moins de 0,1 % en poids de silice.

5. Procédé selon l'une quelconque des revendications 1 à 4, caractérisé en ce que le chlorométhane supérieur est le tétrachlorure de carbone et qu'il est déchloré principalement en chloroforme.

6. Procédé selon l'une quelconque des revendications 1 à 5, caractérisé en ce que la teneur en métal est de 0,01 % à 5 % du poids total du catalyseur.

7. Procédé selon l'une quelconque des revendications 1 à 6, caractérisé en ce que le métal est le platine.

8. Procédé selon l'une quelconque des revendications 1 à 7, caractérisé en ce que le rapport molaire entre l'hydrogène et le chlorométhane supérieur est de 1 à 16.

9. Procédé selon l'une quelconque des revendications 1 à 8, caractérisé en ce que les réactifs sont dilués par du méthane.

10. Procédé selon l'une quelconque des revendications 1 à 9, caractérisé en ce que la température de réaction est d'environ 50 °C à environ 200 °C.

## Claims

1. Process for the dechlorination of higher chloromethanes in the gas phase, using hydrogen, in the presence of a hydrogenation catalyst consisting essentially of at least one metal chosen from the metals of group VIII and group IB of the Periodic Table of the Elements deposited on an alumina, characterised in that the alumina has a specific surface of less than approximately 100 m²/g and in that the dechlorination is carried out substantially in the absence of oxygen.

2. Process according to Claim 1, characterised in that the specific surface of the alumina is from approximately 5 m²/g to approximately 90 m²/g.

3. Process according to Claim 1 or 2, characterised in that the alumina contains no more than 2% by weight of metal oxides other than Al₂O₃.

4. Process according to any one of Claims 1 to 3, characterised in that the alumina contains less than approximately 0.1 % by weight of silica.

5. Process according to any one of Claims 1 to 4, characterised in that the higher chloromethane is carbon tetrachloride and in that it is dechlorinated mainly to chloroform.

6. Process according to any one of Claims 1 to 5, characterised in that the metal content is from approximately 0.01 % to approximately 5 % of the total weight of the catalyst.

7. Process according to any one of Claims 1 to 6, characterised in that the metal is platinum.

8. Process according to any one of Claims 1 to 7, characterised in that the molar ratio of hydrogen to the higher chloromethane is from approximately 1 to approximately 16.

9. Process according to any one of Claims 1 to 8, characterised in that the reactants are diluted with methane.

10. Process according to any one of Claims 1 to 9, characterised in that the reaction temperature is from approximately 50°C to approximately 200°C.

## Patentansprüche

1. Verfahren zur Dechlorierung von höheren Chlormethanen in der Gasphase mit der Hilfe von Wasserstoff in Gegenwart eines Hydrierungskatalysators, bestehend im wesentlichen aus mindestens einem Metall, ausgewählt aus den Metallen der Gruppe VIII und der Gruppe IB des Periodensystems der Elemente, abgeschieden auf einem Aluminiumoxid, dadurch gekennzeichnet, daß das Aluminiumoxid eine spezifische Oberfläche von weniger als 100 m²/g besitzt und daß die Dechlorierung in der im wesentlichen Abwesenheit von Sauerstoff ausgeführt wird.

2. Verfahren nach Anspruch 1, dadurch gekennzeichnet, daß die spezifische Oberfläche des Aluminiumoxids von 5 m²/g bis 90 m²/g ist.

3. Verfahren nach Anspruch 1 oder 2, dadurch gekennzeichnet, daß das Aluminiumoxid nicht mehr als 2 Gew.-% andere Metalloxide als Al₂O₃ enthält.

4. Verfahren nach einem der Ansprüche 1 bis 3, dadurch gekennzeichnet, daß das Aluminiumoxid weniger als 0,1 Gew.-% Siliziumdioxid enthält.

5. Verfahren nach einem der Ansprüche 1 bis 4, dadurch gekennzeichnet, daß das höhere Chlormethan Tetrachlorkohlenstoff ist und daß es hauptsächlich zu Chloroform dechloriert wird.

6. Verfahren nach einem der Ansprüche 1 bis 5, dadurch gekennzeichnet, daß der Gehalt an Metall von 0,01 Gew.-% bis 5 Gew.-% des Gesamtgewichts des Katalysators ist.

7. Verfahren nach einem der Ansprüche 1 bis 6, dadurch gekennzeichnet, daß das Metall Platin ist.

8. Verfahren nach einem der Ansprüche 1 bis 7, dadurch gekennzeichnet, daß das molare Verhältnis von Wasserstoff zu dem höheren Chlormethan von 1 bis 16 ist.

9. Verfahren nach einem der Ansprüche 1 bis 8, dadurch gekennzeichnet, daß die Reaktanten mit Methan verdünnt sind.

10. Verfahren nach einem der Ansprüche 1 bis 9, dadurch gekennzeichnet, daß die Reaktionstemperatur von etwa 50°C bis etwa 200°C ist.
